# EUROPEAN PATENT APPLICATION

(11) **EP 4 643 890 A1**
(43) Date of publication of application: **05.11.2025**
(21) Application number: 23911714.6
(22) Date of filing: 13.12.2023
(51) Int. Cl.: A61K 49/00, A61K 9/10, A61K 47/26

(54) **TISSUE-MARKING AGENT CONTAINING FLUORESCENT DYE-LABELLED PARTICLES**

(30) Priority: 28.12.2022 JP 2022211044
(71) Applicant: National Institute for Materials Science, Tsukuba-shi, Ibaraki 305-0047 (JP); University of Tsukuba, Ibaraki 305-8577 (JP)
(72) Inventor: YOSHITOMI Toru, Tsukuba-shi, Ibaraki 305-0047 (JP); CHEN Guoping, Tsukuba-shi, Ibaraki 305-0047 (JP); KAWAZOE Naoki, Tsukuba-shi, Ibaraki 305-0047 (JP); ODA Tatsuya, Tsukuba-shi, Ibaraki 305-8577 (JP); ENOMOTO Tsuyoshi, Tsukuba-shi, Ibaraki 305-8577 (JP); OHARA Yusuke, Tsukuba-shi, Ibaraki 305-8577 (JP); OWADA Yohei, Tsukuba-shi, Ibaraki 305-8577 (JP); FURUYA Kinji, Tsukuba-shi, Ibaraki 305-8577 (JP)
(74) Representative: J A Kemp LLP
(86) International application number: PCT/JP2023/044604
(87) International publication number: WO 2024/142933

(57) **Abstract**

Provided is a tissue-marking agent that improves the dispersion stability of fine particles labelled with a fluorescent dye in an aqueous medium.

The present invention involves dispersing fine particles labelled with a fluorescent dye in an aqueous medium containing a water-soluble viscous substance.

## Description

### TECHNICAL FIELD

The present invention relates to a tissue-marking agent containing fluorescent dye-labelled particles, and relates to a tissue-marking agent containing fluorescent dye-labelled particles in a specific aqueous medium.

### BACKGROUND ART

In resection surgery of cancer tissue, how much normal tissue can remain is important for maintaining a normal function of organs after surgery. Therefore, in order to avoid unnecessary tissue resection, a tumor site is marked before surgery and then an operation is performed. Also, in particular, gastrointestinal cancer is usually present inside the gastrointestinal tract and there is a need for marking methods that can recognize a tumor site from outside the gastrointestinal tract.

Conventionally, when surgery for gastrointestinal cancer is performed, ink is injected into a tumor site using an endoscope (tattooing method) before surgery or a clip is placed to mark the tumor site.

However, marking with tattooing not only has problems of being difficult to visually recognize because it is not a fluorescent agent, but also of being difficult to determine a boundary between a tumor site and normal tissue due to diffusion of the ink. In addition, the clip cannot be used in surgery using a laparoscope, and even in the case of laparotomy, it may be difficult to palpate from a serosal surface (outside the gastrointestinal tract).

In recent years, endoscopic clips and the like using a phosphor have been developed, but there is a problem that a clip get caught in an automatic suture device when attempting to excise tissue with the automatic suture device (Non-Patent Literature 1).

For such problems of the tattooing method and the clipping method, use of indocyanine green as a tissue-marking agent has been studied. However, since indocyanine green is a low molecular weight compound, it rapidly diffuses even when locally administered, and does not sufficiently function as a tissue-marking agent (Non-Patent Literature 2 and 3).

In addition, it has been reported that tissue was marked using hollow silica nanoparticles with indocyanine green (ICG) electrostatically adsorbed on the surfaces thereof (Non-Patent Literature 4). However, in this marking agent, silica nanoparticles with ICG adsorbed thereon are only dispersed in water, and no consideration is given to dispersion stability. In addition, in this literature, it is described that marking by silica nanoparticles with ICG electrostatically adsorbed thereon can be confirmed for up to about 12 days, and there is a demand for marking for a longer period of time.

### CITATION LIST

### NON-PATENT LITERATURE

NON-PATENT LITERATURE 1: Narihiro et al., International Journal of Surgery
NON-PATENT LITERATURE 2: Yoshiaki Ozawa, Masahiko Murakami, (2020), PREOPERATIVE MARKING METHOD IN LAPAROSCOPIC RECTAL CANCER SURGERY: -INDIA INK TATTOOING VS NEAR-INFRARED (NIR) FLUORESCENCE METHOD-, The Showa University Journal of Medical Sciences 80.1:1-6.
NON-PATENT LITERATURE 3: Tetsuta Satoyoshi et al, Surgical Endoscopy (2021) 35:763-769
NON-PATENT LITERATURE 4: Adrian Garcia Badaracco et al, Indocyanine green modified silica shells for colon tumor marking, Appl Surf Sci. 2020 January 1; 499: doi:10.1016/j.apsusc.2019.143885

### SUMMARY OF INVENTION

### TECHNICAL PROBLEM

The present inventors have studied a biological tissue-marking agent containing fine particles labelled with a fluorescent dye, and newly found a problem that when fine particles labelled with a fluorescent dye are simply dispersed in an aqueous medium, dispersion stability is low, resulting in a concentration gradient within a local injection needle, making it difficult to administer an appropriate amount of a tissue-marking agent, and in some cases, the needle becomes blocked.

The present invention is to solve this problem, that is, to provide a tissue-marking agent that improves the dispersion stability of fine particles labelled with a fluorescent dye in an aqueous medium.

### SOLUTION TO PROBLEM

The present inventors have found that when dispersing fine particles labelled with a fluorescent dye in an aqueous medium containing a water-soluble viscous substance, dispersion stability of the particles is improved and have completed the present invention.

That is, an embodiment of the present invention is as follows.
[1] A tissue-marking agent (a composition for marking tissue) comprising fine particles labelled with a fluorescent dye in an aqueous medium, wherein the fine particles labelled with the fluorescent dye are dispersed in the aqueous medium in which a water-soluble viscous substance is dissolved.
[2] The tissue-marking agent according to [1], wherein the water-soluble viscous substance comprises a polyhydric alcohol, a polyether or a salt thereof, a water-soluble protein, or a polysaccharide or a salt thereof.
[3] The tissue-marking agent according to [2], wherein the water-soluble viscous substance comprises a polysaccharide or a salt thereof.
[4] The tissue-marking agent according to any one of [1] to [3], wherein the fluorescent dye is covalently bonded to the fine particles.
[5] The tissue-marking agent according to any one of [1] to [4], wherein a pH of the tissue-marking agent is in a range of 5.0 to 8.0.
[6] The tissue-marking agent according to any one of [3] to [5], wherein the polysaccharide is at least one selected from the group consisting of hyaluronic acid, alginic acid, guar gum, xanthan gum, acacia gum, pullulan, dextran, and agarose, carrageenan, pectin, galactomannan, xanthan gum, gellan gum, curdlan, carboxymethyl cellulose, chitin/chitosan, and carrageenan.
[7] The tissue-marking agent according to [6], wherein the polysaccharide comprises or is alginic acid, dextran, and/or hyaluronic acid.
[8] The tissue-marking agent according to any one of [1] to [7], wherein the fluorescent dye is selected from the group consisting of indocyanine green, coumarin, rhodamine, xanthene, porphyrin, fluorescamine, fluorescein, and derivatives thereof.
[9] The tissue-marking agent according to [8], wherein the fluorescent dye is indocyanine green or a derivative thereof.
[10] The tissue-marking agent according to any one of [1] to [9], wherein the fine particles are selected from the group consisting of silica particles, polyester particles, polyurethane particles, silicon particles, polyethylene particles, polypropylene particles, polystyrene particles, polymethacrylate particles, titanium alloy particles, hydroxyapatite particles, cellulose particles, silk particles, alumina particles, zirconia particles, and titania particles.
[11] The tissue-marking agent according to any one of [1] to [10], wherein the fine particles have an average particle diameter in a range of 1 nm or more to 2 µm or less.
[12] The tissue-marking agent according to any one of [1] to [11], wherein the dispersion medium is at least one selected from the group consisting of physiological saline, phosphate buffer, acetate buffer, citrate buffer, prolamin buffer, carbon buffer, and purified water.
[13] The tissue-marking agent according to any one of [1] to [12], wherein a concentration of the fine particles in the dispersion medium is in a range of 0.01 mg/mL or more to 1,000 mg/mL or less.
[14] The tissue-marking agent according to any one of [1] to [13], wherein a concentration of the water-soluble viscous substance in the dispersion medium is in a range of 0.001 mass% or more to 20 mass% or less.
[15] The tissue-marking agent according to any one of [1] to [14] for marking a localized area of a gastrointestinal tract.
[16] Use of an aqueous composition in which fine particles labelled with a fluorescent dye are dispersed in an aqueous medium in which a water-soluble viscous substance is dissolved, for preparing a tissue-marking agent.
[17] The use according to [16], wherein the water-soluble viscous substance comprises a polyhydric alcohol, a polyether or a salt thereof, a water-soluble protein, or a polysaccharide or a salt thereof.
[18] The use according to [16], wherein the water-soluble viscous substance comprises a polysaccharide or a salt thereof.
[19] The use according to any one of [16] to [18], wherein the fluorescent dye is covalently bonded to the fine particles.
[20] The use according to any one of [16] to [19], wherein a pH of the tissue-marking agent is in a range of 5.0 to 8.0.
[21] The use according to any one of [18] to [20], wherein the polysaccharide is at least one selected from the group consisting of hyaluronic acid, alginic acid, guar gum, xanthan gum, acacia gum, pullulan, dextran, and agarose, carrageenan, pectin, galactomannan, xanthan gum, gellan gum, curdlan, carboxymethyl cellulose, chitin/chitosan, and carrageenan.
[22] The use according to [21], wherein the polysaccharide comprises or is alginic acid, dextran, and/or hyaluronic acid.
[23] The use according to any one of [16] to [22], wherein the fluorescent dye is selected from the group consisting of indocyanine green, coumarin, rhodamine, xanthene, porphyrin, fluorescamine, and derivatives thereof.
[24] The use according to [23], wherein the fluorescent dye is indocyanine green or a derivative thereof.
[25] The use according to any one of [16] to [24], wherein the fine particles are selected from the group consisting of silica particles, polyester particles, polyurethane particles, silicon particles, polyethylene particles, polypropylene particles, polystyrene particles, polymethacrylate particles, titanium alloy particles, hydroxyapatite particles, cellulose particles, silk particles, alumina particles, zirconia particles, and titania particles.
[26] The use according to any one of [16] to [25], wherein the fine particles have an average particle diameter in a range of 1 nm or more to 2 µm or less.
[27] The use according to any one of [16] to [26], wherein the dispersion medium is at least one selected from the group consisting of physiological saline, phosphate buffer, acetate buffer, citrate buffer, prolamin buffer, carbon buffer, and purified water.
[28] The use according to any one of [16] to [27], wherein a concentration of the fine particles in the dispersion medium is in a range of 0.01 mg/mL or more to 1,000 mg/mL or less.
[29] The use according to any one of [16] to [28], wherein a concentration of the water-soluble viscous substance in the dispersion medium is in a range of 0.001 mass% or more to 20 mass% or less.
[30] The use according to any one of [16] to [29], wherein the tissue-marking agent is used for marking a localized area of a gastrointestinal tract.
[31] An aqueous composition for use in marking tissue, wherein fine particles labelled with a fluorescent dye are dispersed in an aqueous medium in which a water-soluble viscous substance is dissolved.
[32] The aqueous composition for use according to [31], wherein the water-soluble viscous substance comprises a polyhydric alcohol, a polyether or a salt thereof, a water-soluble protein, or a polysaccharide or a salt thereof.
[33] The aqueous composition for use according to [31], wherein the water-soluble viscous substance comprises a polysaccharide or a salt thereof.
[34] The aqueous composition for use according to any one of [31] to [33], wherein the fluorescent dye is covalently bonded to the fine particles.
[35] The aqueous composition for use according to any one of [31] to [34], wherein a pH of the aqueous composition is in a range of 5.0 to 8.0.
[36] The aqueous composition for use according to any one of [31] to [35], wherein the polysaccharide is at least one selected from the group consisting of hyaluronic acid, alginic acid, guar gum, xanthan gum, acacia gum, pullulan, dextran, and agarose, carrageenan, pectin, galactomannan, xanthan gum, gellan gum, curdlan, carboxymethyl cellulose, chitin/chitosan, and carrageenan.
[37] The aqueous composition for use according to [36], wherein the polysaccharide comprises or is alginic acid, dextran, and/or hyaluronic acid.
[38] The aqueous composition for use according to any one of [31] to [37], wherein the fluorescent dye is selected from the group consisting of indocyanine green, coumarin, rhodamine, xanthene, porphyrin, fluorescamine, and derivatives thereof.
[39] The aqueous composition for use according to [38], wherein the fluorescent dye is indocyanine green or a derivative thereof.
[40] The aqueous composition for use according to any one of [31] to [39], wherein the fine particles are selected from the group consisting of silica particles, polyester particles, polyurethane particles, silicon particles, polyethylene particles, polypropylene particles, polystyrene particles, polymethacrylate particles, titanium alloy particles, hydroxyapatite particles, cellulose particles, silk particles, alumina particles, zirconia particles, and titania particles.
[41] The aqueous composition for use according to any one of [31] to [40], wherein the fine particles have an average particle diameter in a range of 1 nm or more to 2 µm or less.
[42] The aqueous composition for use according to any one of [31] to [41], wherein the dispersion medium is at least one selected from the group consisting of physiological saline, phosphate buffer, acetate buffer, citrate buffer, prolamin buffer, carbon buffer, and purified water.
[43] The aqueous composition for use according to any one of [31] to [42], wherein a concentration of the fine particles in the dispersion medium is in a range of 0.01 mg/mL or more to 1,000 mg/mL or less.
[44] The aqueous composition for use according to any one of [31] to [43], wherein a concentration of the water-soluble viscous substance in the dispersion medium is in a range of 0.001 mass% or more to 20 mass% or less.
[45] The aqueous composition for use according to any one of [31] to [44], wherein the tissue-marking agent is used for marking a localized area of a gastrointestinal tract.
[46] A tissue-marking method comprising: topically administering, for example, injecting the tissue-marking agent according to any of [1] to [15] or the aqueous composition according to any of [31] to [45] to a target site of tissue.
[47] The tissue-marking agent according to any of [1] to [15] or the aqueous composition according to any of [31] to [45], comprising an antioxidant.
[48] The tissue-marking agent or the aqueous composition according to [47], comprising the antioxidant at 0.02 mg/mL to 10 mg/mL.
[49] The use according to any one of [16] to [30], wherein the tissue-marking agent comprises an antioxidant.
[50] The use according to [49], wherein the tissue-marking agent comprises the antioxidant at 0.02 mg/mL to 10 mg/mL.

### ADVANTAGEOUS EFFECTS OF INVENTION

When dispersing fine particles labelled with a fluorescent dye in an aqueous medium containing a water-soluble viscous substance, dispersion stability of the particles is improved, thereby allowing administration of an appropriate amount of tissue-marking agent. In addition, it is possible to prevent a local injection needle from being blocked.

### BRIEF DESCRIPTION OF DRAWINGS

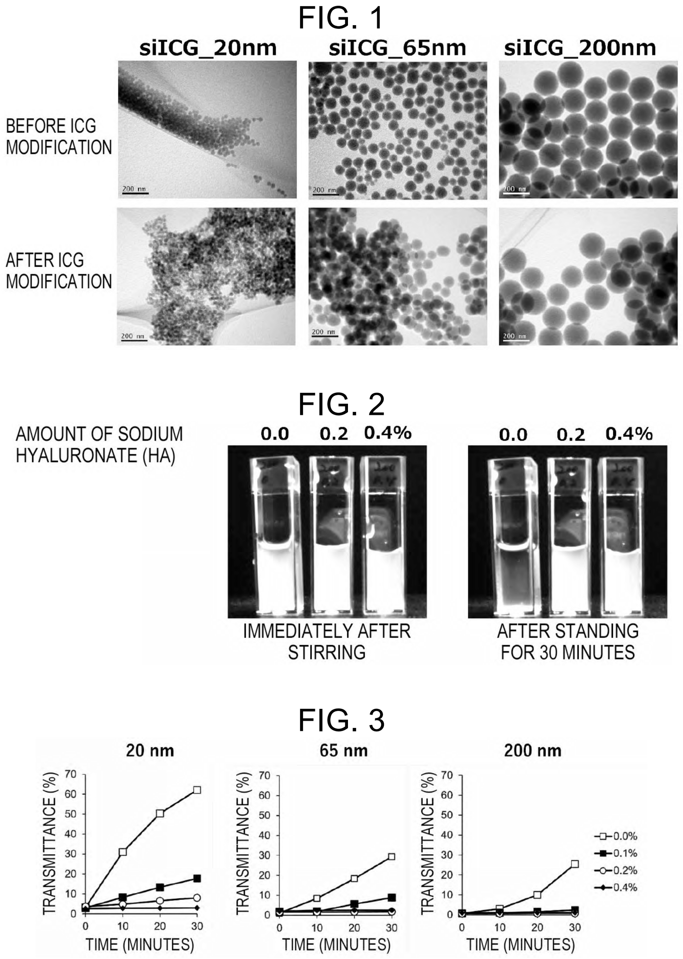

FIG. 1 shows an electron micrograph of silica nanoparticles or indocyanine green-bound silica nanoparticles (siICG) having a particle diameter of 20, 65, or 200 nm.
FIG. 2 shows a dispersion stability of siICG in a case where siICG having a particle diameter of 200 nm is dispersed at a concentration of 5 mg/mL in a phosphate buffer containing 0, 0.2, or 0.4 mass% sodium hyaluronate.
FIG. 3 shows a temporal change in transmittance of light having a wavelength of 500 nm in a case where siICG having a particle diameter of 20, 65, or 200 nm is dispersed at a concentration of 5 mg/mL in a phosphate buffer containing 0, 0.1, 0.2, or 0.4 mass% sodium hyaluronate.

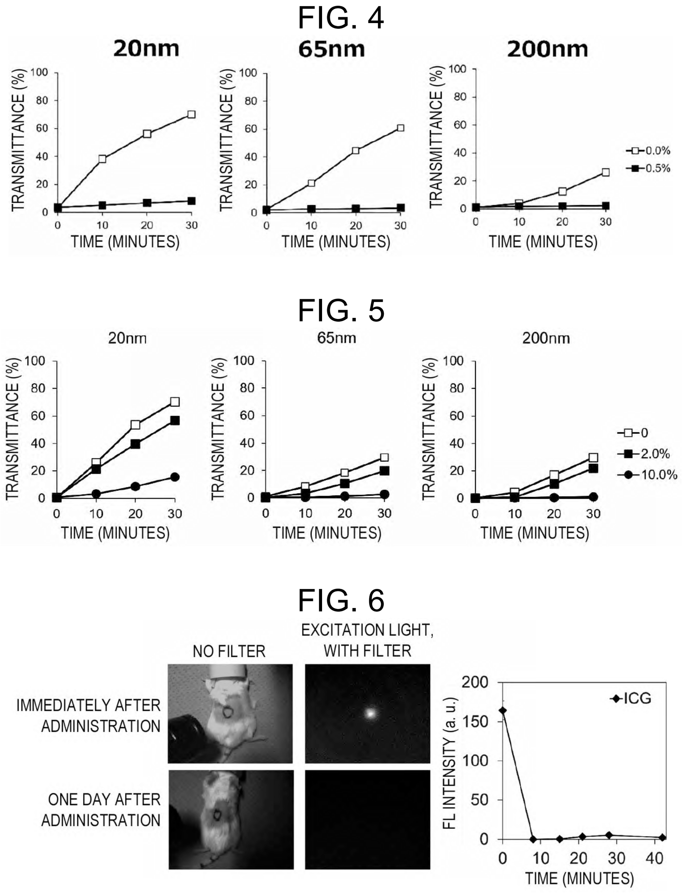

FIG. 4 shows a temporal change in transmittance of light having a wavelength of 500 nm in a case where siICG having a particle diameter of 20, 65, or 200 nm is dispersed at a concentration of 5 mg/mL in a phosphate buffer containing 0 or 0.5 mass% sodium alginate.
FIG. 5 shows a temporal change in transmittance of light having a wavelength of 500 nm in a case where siICG having a particle diameter of 20, 65, or 200 nm is dispersed at a concentration of 5 mg/mL in a phosphate buffer containing 0, 2, or 10 mass% dextran.
FIG. 6 shows a result of subcutaneously administering low molecular indocyanine green to the back of a mouse. Even one day after the administration, no low molecular indocyanine green remained at an administration site.

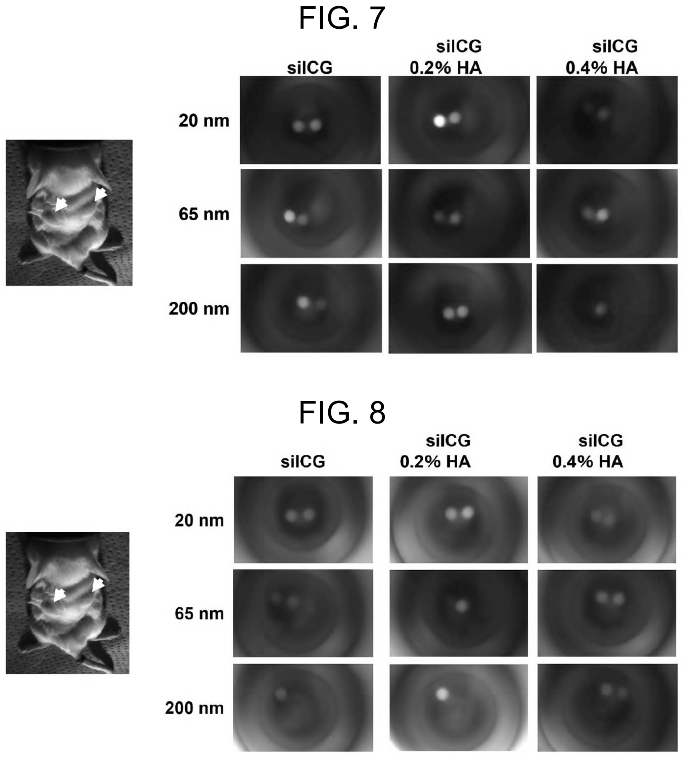

FIG. 7 is a photograph obtained by dispersing siICG having a particle diameter of 20, 65, or 200 nm in a phosphate buffer containing 0, 0.2, or 0.4 mass% sodium hyaluronate at a concentration of 10 mg/mL, subcutaneously administering the siICG solution to a mouse, and then performing imaging with a near-infrared camera 28 days after the administration. Even 28 days after the administration, siICG remained at the administration site and exhibited strong near-infrared emission.
FIG. 8 is a photograph obtained by dispersing siICG having a particle diameter of 20, 65, or 200 nm in a phosphate buffer containing 0, 0.2, or 0.4 mass% sodium hyaluronate at a concentration of 10 mg/mL, subcutaneously administering the siICG solution to a mouse, and then performing imaging with a near-infrared camera 56 days after the administration. Even 56 days after the administration, siICG remained at the administration site and exhibited strong near-infrared emission.

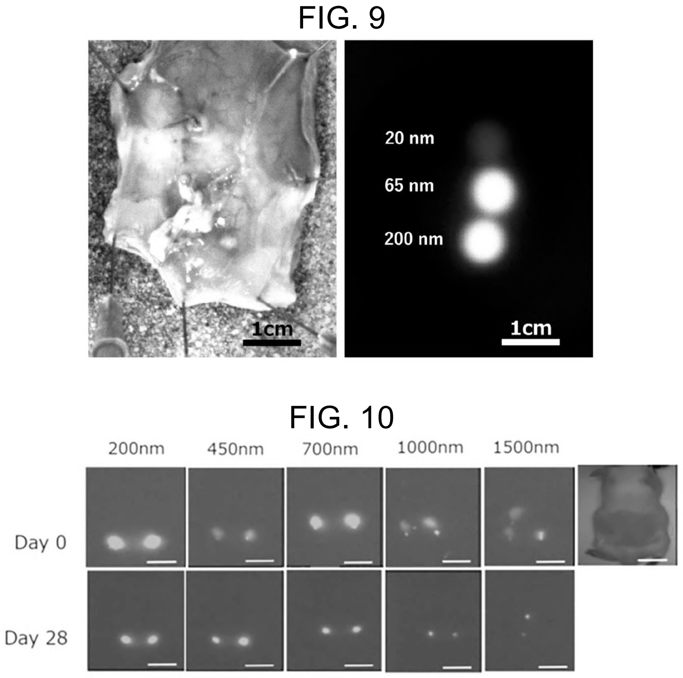

FIG. 9 is a photograph obtained by dispersing siICG having a particle diameter of 20, 65, or 200 nm in a phosphate buffer containing 0.2 mass% sodium hyaluronate at a concentration of 10 mg/mL, respectively, subcutaneously administering the siICG solution to a gastric submucosa of a rabbit, and then performing imaging with a near-infrared camera 21 days after the administration. An excitation light intensity of 2 was used. Even 21 days after the administration, 20, 65, or 200 nm siICG all remained at the administration site and exhibited strong near-infrared emission.
FIG. 10 is a photograph obtained by dispersing siICG having a particle diameter of 200, 450, 700, 1,000, or 1,500 nm in a phosphate buffer containing 0.2 mass% sodium hyaluronate at a concentration of 10 mg/mL, respectively, subcutaneously administering the siICG solution to a mouse, and then performing imaging with a near-infrared camera 28 days after the administration. An excitation light intensity of 2 was used. The photograph at the upper right end is taken to confirm a place where the mouse is installed. Mice were placed in approximately similar positions for all samples. Even 28 days after the administration, 200, 450, 700, 1,000, or 1,500 nm siICG all remained at the administration site and exhibited near-infrared emission.

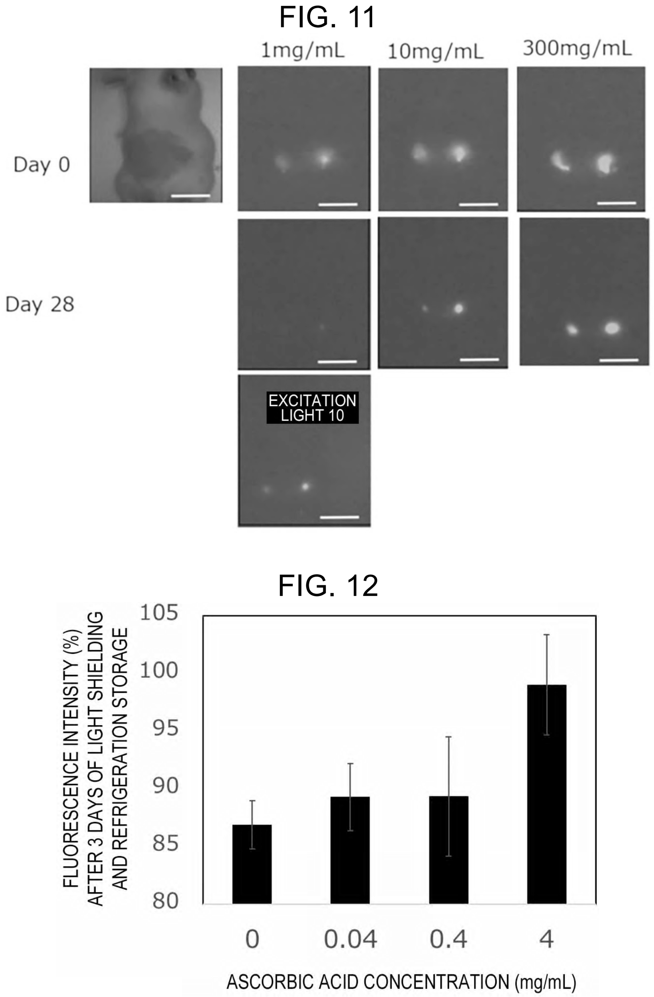

FIG. 11 is a photograph obtained by dispersing siICG having a particle diameter of 200 nm in a phosphate buffer containing 0.2 mass% sodium hyaluronate at concentrations of 1, 10, 300 mg/mL, respectively, subcutaneously administering the siICG solution at each concentration to a mouse, and then performing imaging with a near-infrared camera 28 days after the administration. The photograph at the upper left end is taken to confirm a place where the mouse is installed. Mice were placed in approximately similar positions for all samples. The photographs were basically taken with the excitation light intensity of 2, but the photograph at the bottom of siICG of 1 mg/mL was taken with the excitation light intensity increased from 2 to 10. Even 28 days after the administration, siICG having a concentration of 1, 10, or 300 mg/mL all remained at the administration site and exhibited near-infrared emission.
FIG. 12 is a graph showing fluorescence intensity when a siICG solution prepared by dispersing siICG having a particle diameter of 200 nm in a phosphate buffer containing 0.2 mass% sodium hyaluronate at a concentration of 10 mg/mL and adding ascorbic acid as an antioxidant at a concentration of 0.04, 0.4 or 4 mg/ml or not was stored in a light-shielded manner at 4°C for 3 days and then irradiated with near-infrared light. The fluorescence intensity is indicated as a fluorescence intensity ratio with respect to the fluorescence intensity immediately before light-shielding storage set as 100%. When adding the ascorbic acid, a decrease in the fluorescence intensity of indocyanine green was suppressed.

### DESCRIPTION OF EMBODIMENTS

Embodiments of the present invention will be described in detail. However, the present invention is not limited to the following embodiments.

### <Tissue-marking Agent of Present Invention>

In the present specification, a "tissue-marking agent" means an agent used for marking tissue in surgery or the like, and the tissue-marking agent of the present invention includes fine particles labelled with a fluorescent dye, a water-soluble viscous substance, and a dispersion medium containing at least water, and the fine particles labelled with the fluorescent dye are dispersed in an aqueous viscous medium containing the water-soluble viscous substance, typically in which the water-soluble viscous substance is dissolved.

The fluorescent dye is not particularly limited, and examples thereof include indocyanine green, coumarin, rhodamine, xanthene, porphyrin, fluorescamine, fluorescein, and derivatives thereof. The fluorescent dyes may be used in combination of two or more kinds thereof, and for example, it is also possible to use a mixture of particles in which two or more kinds of fluorescent dyes are adsorbed or bonded to particles having different particle diameters or different materials, respectively.

Since a near-infrared camera is used in a surgical region or a near-infrared region can be observed with high sensitivity without any background signal, a fluorescent dye that emits light in the near-infrared region such as indocyanine green or a derivative thereof is preferable.

The fine particles are not particularly limited, and fine particles made of a biocompatible material are preferable. Also, the fine particles may be biodegradable particles or non-biodegradable particles, and are more preferably non-biodegradable particles from the viewpoint of maintaining the tissue-marking agent in vivo for a long period of time. Examples of the fine particles are not particularly limited, and for example, include silica particles, polyester particles, polyurethane particles, silicon particles, polyethylene particles, polypropylene particles, polystyrene particles, polymethacrylate particles, titanium alloy particles, hydroxyapatite particles, cellulose particles, silk particles, alumina particles, zirconia particles, and titania particles. These particles may be used in combination of two or more kinds thereof, and for example, a mixture of two or more kinds of particles may be used.

In the fine particles labelled with the fluorescent dye, the fluorescent dye is incorporated into the fine particles, or bonded to or adsorbed to the fine particles on surfaces of the fine particles or therein, thereby suppressing rapid diffusion that occurs in a case where the fluorescent dye is topically administered alone. In the present specification, "bond" means a chemically bonded state, and "adsorption" means a physically proximate state by physical interaction, chemical interaction, or both thereof. Examples of the "bond" include a covalent bond, an ionic bond, a hydrogen bond, and the like, and examples of the "adsorption" include a physically proximate state due to, for example, electrostatic interaction, affinity, metal coordination, physical adsorption, host-guest interaction, hydrophobic interaction, pi stacking interaction, van der Waals force, dipole-dipole interaction, and the like.

Depending on a kind of interaction between the fluorescent dye and the fine particles, diffusivity of the fluorescent dye after injection of the tissue-marking agent into tissue may vary. In order to prevent diffusion of the fluorescent dye and to enable marking of a target site clearly and accurately for a long period of time, the fluorescent dye is preferably covalently bonded to the fine particles.

An average particle diameter of the fine particles is not particularly limited, and can be usually in the range of 1 nm or more to 2 µm or less. However, as demonstrated in Examples to be described later, from the viewpoint of dispersion stability and fluorescence signal intensity, the average particle diameter of the fine particles is preferably in the range of 10 nm or more to 1.8 µm or less, preferably in the range of 15 nm or more to 1.5 µm or less, more preferably in the range of 30 nm or more to 1 µmnm or less, still more preferably in the range of 40 nm or more to 700 nm or less, and still further preferably in the range of 50 nm or more to 500 nm or less. A particularly preferable range is 65 nm or more to 300 nm or less. In the present specification, the "average particle diameter" is a value determined by randomly selecting photographs of particles observed with a transmission electron microscope (for example, JEM-2100F, manufactured by JEOL Ltd.), measuring the particle diameters (major diameters) of 100 particles using Image J (ver. 1.53k; open source, public domain image processing software), and averaging the particle diameters.

The amount of the fluorescent dye in the fine particles labelled with the fluorescent dye can be in the range of 0.01 to 5.0 µg, preferably in the range of 0.1 to 2.5 µg, and more preferably in the range of 0.25 to 1.2 µg, per 1 mg of the fine particles. It is particularly preferably in the range of 0.5 to 0.6 µg.

In the present invention, the fine particles labelled with the fluorescent dye described above are dispersed in an aqueous medium containing a water-soluble viscous substance. When dispersing the above-described fine particles labelled with the fluorescent dye in such a dispersion medium, the dispersion stability of the fine particles is improved, and a uniform dispersion liquid for a long time is obtained.

The dispersion medium is typically an aqueous dispersion medium, and an aqueous dispersion medium whose safety is confirmed when applied to a living body is preferable. Examples of the aqueous dispersion medium include physiological saline, phosphate buffer, acetate buffer, citrate buffer, prolamin buffer, carbon buffer, and purified water. The dispersion medium can be used singly or in combination of plural kinds thereof. In addition, various buffer agents may be contained, but it is preferable to contain no basic substance.

The water-soluble viscous substance is a water-soluble substance capable of increasing the viscosity of a dispersion medium, and is usually a substance that can be dissolved in a dispersion medium. Such a substance is usually a polymer compound having a plurality of hydrophilic functional groups such as a hydroxyl group, and preferably has a molecular weight (Mw) of 10,000 to 1 million and more preferably has a molecular weight (Mw) of 10,000 to 100,000 in order to provide an appropriate viscosity for a dispersion medium. Preferred examples of the water-soluble viscous substance may include a polyhydric alcohol, a polyether or a salt thereof, a water-soluble protein, and a polysaccharide or a salt thereof, and among these, a biocompatible water-soluble viscous substance is more preferable. In particular, polysaccharide or a salt thereof exhibits excellent dispersion stability of fine particles as demonstrated in Examples to be described later, and is a particularly preferable water-soluble viscous substance from the viewpoint of dispersion stability of fine particles and biocompatibility.

Examples of a polyhydric alcohol include ethylene glycol and glycerin. Also, the polyhydric alcohol is preferably a polyhydric alcohol having a molecular weight (Mw) of 50 to 1,000 from the viewpoint of providing an appropriate viscosity to a dispersion medium.

Examples of the polyether include polyethylene glycol. In addition, a polyether having a molecular weight (Mw) of 1,000 to 1 million is preferable from the viewpoint of providing an appropriate viscosity to a dispersion medium.

Examples of the water-soluble protein include albumin (particularly, human serum albumin), fibrin, and globulin (particularly, human immunoglobulin).

In the present specification, unless otherwise specified, the "molecular weight" means a weight average molecular weight, and the weight average molecular weight means a weight average molecular weight obtained by gel permeation chromatography (GPC).

The polysaccharide is not particularly limited, and examples thereof include hyaluronic acid, alginic acid, guar gum, xanthan gum, acacia gum, pullulan, dextran, and agarose, carrageenan, pectin, galactomannan, xanthan gum, gellan gum, curdlan, carboxymethyl cellulose, chitin/chitosan, and carrageenan. The polysaccharide whose safety when injected into a living body has been established is preferable, and from the viewpoint of dispersion stability of fine particles and biocompatibility, hyaluronic acid, dextran, and alginic acid are more preferable, and hyaluronic acid and alginic acid are particularly preferable.

The polysaccharide may be a salt, and is not particularly limited as long as it is a biocompatible salt. Examples of the salt include inorganic base salts. Examples of the inorganic base salt include salts derived from inorganic bases such as sodium, potassium, lithium, ammonium, calcium, magnesium, iron, zinc, copper, manganese, and aluminum.

The polysaccharide or a salt thereof can be used singly or in combination of plural kinds thereof. The polysaccharide or a salt thereof is usually dissolved in an aqueous medium, and is not adsorbed or bonded to fine particles.

The concentration of the fine particles in the dispersion medium is not particularly limited, can be usually 0.01 mg/mL or more to 1,000 mg/mL or less, and is preferably in the range of 0.1 mg/mL or more to 500 mg/mL or less, more preferably in the range of 1 mg/mL or more to 400 mg/mL or less, still more preferably in the range of 10 mg/mL or more to 300 mg/mL or less, and particularly preferably in the range of 50 mg/mL or more to 300 mg/mL or less.

The concentration of the water-soluble viscous substance in the dispersion medium varies depending on the water-soluble viscous substance to be used, but it can be usually adjusted in the range of 0.001 mass% or more to 20 mass% or less to prepare a dispersion medium having an appropriate viscosity.

In a case of a polyhydric alcohol-containing medium, the concentration of the polyhydric alcohol in the dispersion medium varies depending on the molecular weight and the degree of hydrophilicity of the polyhydric alcohol to be used, and it is preferable to select a concentration in an appropriate range from the viewpoint of solubility and dispersion stability of particles. Usually, the concentration may be selected from the range of 0.01 mass% or more to 20% mass or less, is preferably selected from the range of 0.1 mass% or more to 15 mass% or less, and is particularly preferably 0.2 mass% or more to 10 mass% or less.

In a case of a polyether-containing medium, the concentration of the polyether in the dispersion medium varies depending on the molecular weight and the degree of hydrophilicity of the polyether to be used, and it is preferable to select a concentration in an appropriate range from the viewpoint of solubility and dispersion stability of particles. Usually, the concentration may be selected from the range of 0.01 mass% or more to 20 mass% or less, is preferably selected from the range of 0.1 mass% or more to 15 mass% or less, and is particularly preferably 0.2 mass% or more to 10 mass% or less.

In a case of a water-soluble protein-containing medium, the concentration of the protein in the dispersion medium varies depending on the molecular weight and the degree of hydrophilicity of the protein to be used, and it is preferable to select a concentration in an appropriate range from the viewpoint of solubility and dispersion stability of particles. Usually, the concentration may be selected from the range of 0.01 mass% or more to 20 mass% or less, is preferably selected from the range of 0.1 mass% or more to 15 mass% or less, and is particularly preferably 0.2 mass% or more to 10 mass% or less.

In a case of a medium containing a polysaccharide or a salt thereof, the concentration of the polysaccharide or a salt thereof in the dispersion medium varies depending on the molecular weight and the degree of hydrophilicity of the polysaccharide or a salt thereof to be used, and it is preferable to select a concentration in an appropriate range from the viewpoint of solubility and dispersion stability of particles. Usually, the concentration can be selected from the range of 0.001 mass% or more to 20 mass% or less, and is preferably selected from the range of 0.01 mass% or more to 10 mass% or less. In a case of a medium containing hyaluronic acid or a salt thereof, the concentration of the hyaluronic acid or a salt thereof in the dispersion medium may be usually selected from the range of 0.01 mass% or more to 10 mass% or less, and is preferably 0.1 mass% or more to 5 mass% or less, more preferably 0.15 mass% or more to 1 mass% or less, and particularly preferably 0.15 mass% or more to 0.5 mass% or less. In a case of a medium containing alginic acid or a salt thereof, the concentration of the alginic acid or a salt thereof in the dispersion medium may be usually selected from the range of 0.01 mass% or more to 10 mass% or less, and is preferably 0.1 mass% or more to 5 mass% or less, more preferably 0.15 mass% or more to 1 mass% or less, and particularly preferably 0.2 mass% or more to 0.8 mass% or less. In a case of a medium containing dextran or a salt thereof, the concentration of the dextran or a salt thereof in the dispersion medium may be usually selected from the range of 2.0 mass% or more to 20 mass% or less, and is preferably 3.0 mass% or more to 17 mass% or less, more preferably 5.0 mass% or more to 15 mass% or less, and particularly preferably 8.0 mass% or more to 12.0 mass% or less.

A pH of the tissue-marking agent is preferably in a range of about 5.0 to about 8.0, and more preferably in a range of about 5.5 to about 8.0, about 6.0 to about 8.0, about 6.5 to about 8.0, about 5.0 to about 7.5, about 5.5 to about 7.5, about 6.0 to about 7.5, or about 6.5 to about 7.5. In a case where the pH is in the ranges, not only high safety is achieved without causing inflammation or the like when the tissue-marking agent is topically injected into a living body, but also good solubility of the water-soluble viscous substance in the dispersion medium is maintained, so that the water-soluble viscous substance is hardly adsorbed to or bonded to fine particles.

The tissue-marking agent may optionally include various additives. For example, a dye that does not require a near infrared camera, typically a dye that can be visually confirmed can be added in order to confirm whether administration has been properly performed at the time of administration, or an antioxidant can be added in order to avoid a decrease in the fluorescence intensity of the above-described fluorescent dye (for example, indocyanine green) due to oxidation at the time of storage. Examples of such a dye include indigo carmine, cardio blue, brilliant blue, methylene blue, triidine blue, and trypan blue, and examples of the antioxidant include ascorbic acid, sodium ascorbate, and vitamin E. Also, an amount of the antioxidant such as ascorbic acid or sodium ascorbate may be usually 0.01 mg/mL or more in the tissue-marking agent, and is preferably 0.02 mg/mL to 10 mg/mL, more preferably 0.04 mg/mL to 7 mg/mL, and particularly preferably 2 mg/mL to 6 mg/mL.

### <Method for Producing Tissue-marking Agent of Present Invention>

The tissue-marking agent of the present invention is produced by adding fine particles labelled with a fluorescent dye to a dispersion medium containing a water-soluble viscous substance and stirring the mixture.

The fine particles labelled with a fluorescent dye are produced by labeling the interior or surface of the fine particles with a fluorescent dye by physical interaction, chemical interaction, or both thereof. A method for labeling fine particles with a fluorescent dye is well known in the technical field to which the present invention belongs, and typically, a functional group that causes a physical interaction, a chemical interaction, or both thereof between the fine particles and the fluorescent dye is introduced into one or both of the fine particles and the fluorescent dye, and the fine particles and the fluorescent dye are bonded or adsorbed through the functional group. For example, depending on the fluorescent dye to be used, the surfaces of the fine particles are appropriately modified using a silane coupling agent or the like to change the surface charge, hydrophobicity, hydrophilicity, or the like of the fine particles, or to introduce a reactive functional group into the fine particles. Similarly, if necessary, an appropriate modification such as introduction of a corresponding functional group is also introduced into the fluorescent dye according to the modification introduced into the surfaces of the fine particles. After an appropriate modification is introduced into one or both of the fine particles and the fluorescent dye, the fine particles and the fluorescent dye are brought into contact (react) with each other, so that a desired physical and/or chemical interaction occurs, and the fine particles and the fluorescent dye are bonded or adsorbed to each other. Examples of the functional group that can be introduced into the fine particles and/or the fluorescent dye include an amino group, a carboxyl group, a thiol group, a vinyl group, an epoxy group, a styryl group, a methacrylic group, an acrylic group, a ureido group, a mercapto group, and an isocyanate group.

A method for modifying fine particles using a silane coupling agent is well known in the technical field to which the present invention belongs. A method for modifying alumina particles with a silane coupling agent is described, for example, in Composites Science and Technology 68 (2008) 2965-2975. A method for modifying zirconia particles with a silane coupling agent is described, for example, in Langmuir 2008, 24, 11497-11505. A method for modifying cellulose particles with a silane coupling agent is described, for example, in Journal of Colloid and Interface Science 289 (2005) 249-261. A method for modifying titanium alloy particles and titania particles with a silane coupling agent is described, for example, in Colloids and Surfaces A: Physicochem. Eng. Aspects 413 (2012) 273-279. A method for modifying hydroxyapatite particles with a silane coupling agent is described, for example, in Materials Science and Engineering C 58 (2016) 675-681. A method for modifying polypropylene particles with a silane coupling agent is described, for example, in Polymer composites 32.10 (2011): 1568-1583. A method for modifying polystyrene particles with a silane coupling agent is described, for example, in Chem. Mater. 2002, 14, 1325-1331 and Applied Surface Science 499 (2020) 143885, and also polyester particles, polyurethane particles, silicon particles, polyethylene particles, silk particles, and polymethacrylate particles cay be modified by the methods described in these documents. The contents of these documents are incorporated herein by reference.

### <Applications of Tissue-marking Agent of Present Invention>

The tissue-marking agent of the present invention can be used for marking a localized area of living tissue, and the tissue to be marked is not particularly limited, and examples thereof include cancer, lymph node, gastrointestinal tract, pancreas, mammary gland, skin, lung, kidney, bladder, thyroid, prostate, head and neck, liver, bile duct, peripheral nerve, brain, skeletal muscle, smooth muscle, adipose tissue, and uterus. The tissue-marking agent of the present invention can also be used for marking at a cellular level, and examples of a marking target include tumor cells, a tumor cell mass, a solid tumor, the vicinity of a solid tumor, and a precancerous lesion. In one embodiment of the present invention, taking advantage of the property of being held in a localized area for a long period of time, the tissue-marking agent can also be used for decorating the body by injecting it subcutaneously.

A method for administering the tissue-marking agent of the present invention is not particularly limited as long as it is a method that enables administration to a target site, and examples thereof include submucosal injection, intramuscular injection, intratumoral injection, subcutaneous injection, and intraperitoneal injection.

### Examples

Hereinafter, the present invention will be described in more detail with reference to Examples. However, the present invention is not limited to the following examples.

### 1. Production of Indocyanine Green-Bound Silica Nanoparticles

### 1-1. Synthesis of Amino Group-Modified Silica Nanoparticles

As silica nanoparticles, commercially available silica nanoparticles having three kinds of particle diameters (SNOWTEX, manufactured by Nissan Chemical Corporation, product name: 22 nm; ST-50-T, 65 nm; ST-YL, 200 nm; and ST MP-2040) were used. 0.05 mL of (3-aminopropyl)triethoxysilane, 1.5 mL of aqueous ammonia (28 mass%), and 15 mL of ultrapure water were added to 5 mL (480 mg/mL) of silica nanoparticles having three kinds of particle diameters, and the mixture was vigorously stirred to obtain a mixed solution. Thereafter, the obtained mixed solution was heated at 75°C for 3 hours, placed in a dialysis membrane having a molecular weight cutoff of 3,500, and dialyzed against water for 1 day to purify amino group-modified silica nanoparticles.

### 1-2. Synthesis of Indocyanine Green-Bound Silica Nanoparticles (siICG)

1 µL of a phosphate buffer (200 mM, pH 7.0) was added to 2 mL of the amino group-modified silica nanoparticles obtained after dialysis to adjust the pH, thereby obtaining an aqueous solution in which silica nanoparticles were dispersed. On the other hand, in another container, 0.3 mg of ICG NHS ester (Catalog No. POS1604, manufactured by Funakoshi Co., Ltd.), 30 mg of WSCD-HCl (manufactured by Peptide Laboratories Co., Ltd.), and 23 mg of N-hydroxysuccinimide (manufactured by FUJIFILM Wako Pure Chemical Corporation) were dissolved in 5 mL of pure water. The entire amount of the solution was added to the aqueous solution in which silica nanoparticles were dispersed, and the mixture was reacted at 25°C for 2 hours. Thereafter, the reacted aqueous solution was placed in a dialysis membrane having a molecular weight cutoff of 3,500, first subjected to dialysis against methanol for 3 hours, and then subjected to dialysis against pure water for 1 day to purify indocyanine green-bound silica nanoparticles (siICG). Indocyanine green that is not bound to the silica nanoparticles is separated from the nanoparticles by adding methanol or ethanol to the produced particles, and thus can be removed. On the other hand, since the bound indocyanine green is not desorbed even when methanol is added, it can be confirmed that the indocyanine green is bound to the silica nanoparticles.

After dialysis, freeze-drying was performed to collect siICG particles. The amount of indocyanine green bound per 1 mg of the weight of the collected siICG particles was 0.5 to 0.6 µg. Also, for sterilization, ethylene oxide gas sterilization was performed. Electron micrographs of silica nanoparticles before and after ICG labeling are shown in FIG. 1.

### 2. Preparation of Tissue-Marking Agent (1)

Sodium hyaluronate (manufactured by FUJIFILM Wako Pure Chemical Corporation) was added to 1 mL of a phosphate buffer solution (PBS, manufactured by Nacalai Tesque) to prepare phosphate buffer solutions in which sodium hyaluronate having sodium hyaluronate concentrations of 0.1 mass%, 0.2 mass%, or 0.4 mass%, respectively, was dissolved. 5 mg of the collected siICG powder or 10 mg of the collected siICG powder was added to and stirred with the phosphate buffer solution in which sodium hyaluronate at each concentration was dissolved to prepare a tissue-marking agent. Also, as a comparative example, 5 mg of the collected siICG powder or 10 mg of the collected siICG powder was added to and stirred with the phosphate buffer solution in which sodium hyaluronate was not added to prepare a tissue-marking agent.

The list of tissue-marking agents obtained was as follows.

**[Table 1]**

| | Silica particles | | Sodium hyaluronate | Dispersion medium |
|---|---|---|---|---|
| | Particle diameter (nm) | Amount (mg/mL) | Concentration (mass%) | |
| Example 1 | 20 | 5 | 0.1 | PBS |
| Example 2 | 20 | 5 | 0.2 | PBS |
| Example 3 | 20 | 5 | 0.4 | PBS |
| Comparative Example 1 | 20 | 5 | 0.0 | PBS |
| Example 4 | 65 | 5 | 0.1 | PBS |
| Example 5 | 65 | 5 | 0.2 | PBS |
| Example 6 | 65 | 5 | 0.4 | PBS |
| Comparative Example 2 | 65 | 5 | 0.0 | PBS |
| Example 7 | 200 | 5 | 0.1 | PBS |
| Example 8 | 200 | 5 | 0.2 | PBS |
| Example 9 | 200 | 5 | 0.4 | PBS |
| Comparative Example 3 | 200 | 5 | 0.0 | PBS |
| Example 10 | 20 | 10 | 0.1 | PBS |
| Example 11 | 20 | 10 | 0.2 | PBS |
| Example 12 | 20 | 10 | 0.4 | PBS |
| Comparative Example 4 | 20 | 10 | 0.0 | PBS |
| Example 13 | 65 | 10 | 0.1 | PBS |
| Example 14 | 65 | 10 | 0.2 | PBS |
| Example 15 | 65 | 10 | 0.4 | PBS |
| Comparative Example 5 | 65 | 10 | 0.0 | PBS |
| Example 16 | 200 | 10 | 0.1 | PBS |
| Example 17 | 200 | 10 | 0.2 | PBS |
| Example 18 | 200 | 10 | 0.4 | PBS |
| Comparative Example 6 | 200 | 10 | 0.0 | PBS |

### 3. Preparation of Tissue-Marking Agent (2)

Sodium alginate (manufactured by FUJIFILM Wako Pure Chemical Corporation) or dextran (40kDa, manufactured by FUJIFILM Wako Pure Chemical Corporation) was added to a phosphate buffer solution (PBS, manufactured by Nacalai Tesque) to prepare a phosphate buffer solution in which 0.5 mass% of sodium alginate or 2 mass% or 10 mass% of dextran was dissolved. 5 mg of the collected siICG particles was added to 1 mL of each solution and stirred to prepare a tissue-marking agent. The list of tissue-marking agents obtained was as follows.

**[Table 2]**

| | Silica particles | | Sodium alginate | Dextran | Dispersion medium |
|---|---|---|---|---|---|
| | Particle diameter (nm) | Amount (mg/mL) | Concentration (mass%) | Concentration (mass%) | |
| Example 19 | 20 | 5 | 0.5 | - | PBS |
| Example 20 | 65 | 5 | 0.5 | - | PBS |
| Example 21 | 200 | 5 | 0.5 | - | PBS |
| Example 22 | 20 | 5 | - | 2 | PBS |
| Example 23 | 65 | 5 | - | 2 | PBS |
| Example 24 | 200 | 5 | - | 2 | PBS |
| Example 25 | 20 | 5 | - | 10 | PBS |
| Example 26 | 65 | 5 | - | 10 | PBS |
| Example 27 | 200 | 5 | - | 10 | PBS |

### 4. Evaluation of Dispersion State of siICG in Dispersion Medium

FIG. 2 shows photographs taken immediately after adding and stirring siICG, and photographs taken after allowing a mixture to stand for 30 minutes after stirring, regarding the tissue-marking agents of Comparative Example 1 and Examples 2 and 3 prepared by adding and stirring 5 mg of siICG (particle diameter: 200 nm) in 1 mL of a phosphate buffer solution (PBS) containing 0.0 mass%, 0.2 mass%, or 0.4 mass% sodium hyaluronate. In the tissue-marking agent of Comparative Example 1 in which siICG was dispersed in PBS containing no sodium hyaluronate, siICG precipitated within 30 minutes. On the other hand, in the tissue-marking agents of Examples 2 and 2 in which siICG was dispersed in PBS containing sodium hyaluronate at a concentration of 0.2 mass% or 0.4 mass%, a state in which siICG was dispersed was maintained for 30 minutes or more.

In order to evaluate the dispersion state of siICG in the dispersion medium, the transmittance of the tissue-marking agents of Examples 1 to 9 and Comparative Examples 1 to 3 were measured with a UV-vis spectrophotometer (UV-2600, Shimadzu Corp.) (FIG. 3). When the sedimentation rate was compared among solutions containing siICG having a particle diameter of 20, 65, or 200 nm, the smaller the particle diameter of siICG, the faster the sedimentation rate tended to be. Comparing the tissue-marking agents of Comparative Examples 1 to 3 in which siICG was dispersed in PBS containing no sodium hyaluronate, in a case of the tissue-marking agent of Comparative Example 1 containing siICG having a particle diameter of 20 nm, about 60% of siICG precipitated after 30 minutes, and in a case of the tissue-marking agents of Comparative Examples 2 and 3 containing siICG having a particle diameter of 65 or 200 nm, about 30% of siICG precipitated after 30 minutes. In the tissue-marking agents of Examples 1 to 3 in which the siICG having a particle diameter of 20 nm was dispersed in PBS containing 0.1 mass% or more of sodium hyaluronate, a state in which about 60% of the siICG was dispersed was maintained even after 30 minutes, and in the tissue-marking agents of Examples 2 and 3 in which the siICG was dispersed in PBS containing 0.2 mass% or more of sodium hyaluronate, a state in which most of the siICG was dispersed was maintained even after 30 minutes. In the tissue-marking agents of Examples 4 to 9 in which siICG having a particle diameter of 65 nm or 200 nm was dispersed in PBS containing 0.1 mass% or more of sodium hyaluronate, a state in which most of the siICG was dispersed was maintained even after 30 minutes.

Although not shown in the graph, in the tissue-marking agents of Examples 10 to 18 and Comparative Examples 4 to 6 in which the amount of siICG was 10 mg/mL, the same results as in the tissue-marking agents of Examples 1 to 9 and Comparative Examples 1 to 3 were obtained, respectively.

Next, the dispersion states of siICG were evaluated by comparing the tissue-marking agents of Examples 19 to 21 and Examples 22 to 27 in which siICG was dispersed in PBS containing sodium alginate or dextran with the tissue-marking agent of Comparative Example 1 in which siICG was dispersed in PBS without containing these water-soluble viscous substances.

As shown in FIG. 4, in the tissue-marking agents of Examples 19 to 21 in which siICG having a particle diameter of 20, 65, or 200 nm was dispersed in PBS containing 0.5 mass% sodium alginate, a state in which most of the siICG was dispersed was maintained even after 30 minutes.

In addition, as shown in FIG. 5, in the tissue-marking agents of Examples 22 to 27 in which siICG having a particle diameter of 20, 65, or 200 nm was dispersed in PBS containing 0, 2, or 10 mass% dextran, the dispersion stability was improved as compared with the tissue-marking agent of Comparative Example 1 in which siICG was dispersed in PBS containing no dextran, and in the tissue-marking agents of Examples 25 to 27 in which siICG was dispersed in PBS containing 10 mass% dextran, a state in which most of the siICG was dispersed was maintained even after 30 minutes.

### 5. Topical Administration of Solution Containing Indocyanine Green or siICG Subcutaneously in Mice

The back of ICR mice (female, 5 weeks old) was shaved using a hair clipper. 100 µL of low molecular indocyanine green (Product code: I0535, Tokyo Chemical Industry Co., Ltd.) was injected subcutaneously into the shaved back, and one day later, the administration site was irradiated with an LED light of 780 nm under isoflurane anesthesia, and the local injection site was imaged using a near-infrared digital camera (ORCAspark, Hamamatsu Photonics) (FIG. 6). One day after the administration, no low molecular indocyanine green remained at the administration site.

50 µL each of the tissue-marking agents of Examples 10 to 18 and Comparative Examples 4 to 6 was injected subcutaneously at both sides under the hypoderm of the back of a shaved ICR mouse (female, 5 weeks old). After the injection, 28 days and 56 days later, the administration site was irradiated with an LED light of 780 nm under isoflurane anesthesia, and the local injection site was imaged using a near-infrared digital camera (ORCAspark, Hamamatsu Photonics). As shown in FIGS. 7 and 8, it was confirmed that even in a case where any tissue-marking agent was administered, strong near-infrared emission was detected from the administration site 28 and 56 days after administration, and siICG remained at the administration site.

### 6. Topical Administration to Rabbit Gastric Submucosa

A sedative medetomidine was intramuscularly injected, and the surgical field was shaved using a hair clipper under inhalation anesthesia with isoflurane, and disinfection was performed using isodine. The abdomen was subjected to median incision (about 10 cm), the stomach was exposed to the outside of the abdominal cavity through the incision, and 100 µL of a tissue-marking agent of Example 11, 14, or 17 containing siICG having a particle diameter of 20 nm, 65 nm or 200 nm was topically injected into the gastric wall (near the submucosa layer). After 21 days, the animals were euthanized, the excised stomachs were opened, and the injection site was irradiated with an LED light of 780 nm and the local injection site was imaged using a near-infrared digital camera (ORCAspark, Hamamatsu Photonics). An excitation light intensity of 2 was used. Even 21 days after administration, a fluorescence signal was detected from the tissue-marking agent administration site, and it was confirmed that siICG remained at the administration site (FIG. 9). In addition, the fluorescence signals at the site where the tissue-marking agent containing siICG having a particle diameter of 65 nm or 200 nm was administered were about 2.6 times and about 2.2 times stronger than the fluorescence intensity at the site where the tissue-marking agent containing siICG having a particle diameter of 20 nm was administered. It is suggested that the larger the particle diameter of siICG is, the more the residual property of the tissue-marking agent to the administration site is improved, and this is considered that the diffusion due to the movement of the digestive tract and the diffusion to the mucosal layer are easily suppressed as the particle diameter increases.

### 7. Evaluation of Particle Diameter

Indocyanine green-bound silica nanoparticles (siICG) were produced according to the description of "1. Production of Indocyanine Green-Bound Silica Nanoparticles" except that silica particles having a particle diameter of 200 nm (product name: SNOWTEX, ST MP-2040, manufactured by Nissan Chemical Corporation), silica particles having a particle diameter of 450 nm (product name: SNOWTEX, ST MP-4540M, manufactured by Nissan Chemical Corporation), silica particles having a particle diameter of 700 nm (product name: SS-07, manufactured by Tokuyama Corporation), silica particles having a particle diameter of 1,000 nm (product name: SS-10, manufactured by Tokuyama Corporation), or silica particles having a particle diameter of 1,500 nm (product name: SS-15, manufactured by Tokuyama Corporation) were used, and a tissue-marking agent was prepared according to the description of "2. Preparation of Tissue-Marking Agent (1)" except that 10 mg of the collected siICG was added to 1 mL of a phosphate buffer containing 0.2 mass% sodium hyaluronate and stirred.

According to the description of "5. Topical Administration of Solution Containing Indocyanine Green or siICG Subcutaneously in Mice", 50 µL of each of the prepared tissue-marking agents was injected subcutaneously on both sides under the hypoderm of the back of a shaved ICR mouse (female, 5 weeks old), and immediately after administration (day 0) and 28 days after administration, the administration site was irradiated with an LED light of 780 nm under isoflurane anesthesia, and the local injection site was imaged using a near-infrared digital camera (pde-neo, Hamamatsu Photonics). An excitation light intensity of 2 was used.

Photographs obtained by imaging the administration site of the mouse to which siICG having each particle diameter was administered using a digital camera (pde-neo, Hamamatsu Photonics) immediately after administration (day 0) and 28 days after administration are shown in FIG. 10. An excitation light intensity of 2 was used.

As understood from FIG. 10, the fluorescence intensity per weight of siICG tended to decrease as the particle diameter of siICG increased. However, even in a case of siICG having a particle diameter of 1,500 nm, near-infrared emission was detected after 28 days, and it was confirmed that siICG remained at the administration site.

### 8. Evaluation of Concentration of siICG

Indocyanine green-bound silica nanoparticles (siICG) were produced according to the description of "1. Production of Indocyanine Green-Bound Silica Nanoparticles" except that silica particles having a particle diameter of 200 nm (product name: SNOWTEX, ST MP-2040, manufactured by Nissan Chemical Corporation) were used, and a tissue-marking agent was prepared according to the description of "2. Preparation of Tissue-Marking Agent (1)" except that 1, 10, or 300 mg of the collected siICG was added to 1 mL of a phosphate buffer containing 0.2 mass% sodium hyaluronate and stirred.

According to the description of "5. Topical Administration of Solution Containing Indocyanine Green or siICG Subcutaneously in Mice", 50 µL of the prepared tissue-marking agent at each concentration was injected subcutaneously on both sides under the hypoderm of the back of a shaved ICR mouse (female, 5 weeks old), and immediately after administration (day 0) and 28 days after administration, the administration site was irradiated with an LED light of 780 nm under isoflurane anesthesia, and the local injection site was imaged using a near-infrared digital camera. As the excitation light intensity, basically 2 was used, but in a case where a 1 mg/mL tissue-marking agent was used, excitation light intensities of 2 and 10 were used.

Photographs obtained by imaging a local injection site of the administration site of the mouse to which tissue-marking agent at each concentration was administered using a digital camera immediately after administration (day 0) and 28 days after administration are shown in FIG. 11.

As understood from FIG. 11, the fluorescence intensity tended to increase in a concentration-dependent manner, and near-infrared emission was detected after 28 days in a concentration range of 1 to 300 mg/mL, confirming that siICG remained at the administration site.

### 9. Effect of Suppressing Decrease in Fluorescence Intensity by Addition of Antioxidant

Indocyanine green-bound silica nanoparticles (siICG) were produced according to the description of "1. Production of Indocyanine Green-Bound Silica Nanoparticles" except that silica particles having a particle diameter of 200 nm (product name: SNOWTEX, ST MP-2040, manufactured by Nissan Chemical Corporation) were used, and a tissue-marking agent was prepared according to the description of "2. Preparation of Tissue-Marking Agent (1)" except that 10 mg of the collected siICG was added to 1 mL of a phosphate buffer containing 0.2 mass% sodium hyaluronate and an 0.04, 0.4, or 4 mg of ascorbic acid as an antioxidant was added or not and stirred.

After each prepared tissue-marking agent was stored at 4°C for 3 days in a light-shielding chamber, 100 µL of the tissue-marking agent was transferred to a black 96 well plate, and the fluorescence intensity was measured using a plate reader (Tecan, product name: Trading AG, TECAN).

The fluorescence intensity after light-shielding storage of the tissue-marking agent to which ascorbic acid was not added or to which ascorbic acid was added at each concentration is shown in FIG. 12.

As understood from FIG. 12, it was confirmed that the decrease in the fluorescence intensity of indocyanine green was suppressed by the addition of ascorbic acid, and the decrease in the fluorescence intensity was remarkably suppressed in the tissue-marking agent to which 4 mg of ascorbic acid was added.

## Claims

1. A tissue-marking agent comprising fine particles labelled with a fluorescent dye in an aqueous medium, wherein the fine particles to which the fluorescent dye is adsorbed or bound are dispersed in the aqueous medium in which a water-soluble viscous substance is dissolved.

2. The tissue-marking agent according to claim 1, wherein the water-soluble viscous substance comprises a polyhydric alcohol, a polyether or a salt thereof, a water-soluble protein, or a polysaccharide or a salt thereof.

3. The tissue-marking agent according to claim 2, wherein the water-soluble viscous substance comprises a polysaccharide or a salt thereof.

4. The tissue-marking agent according to any one of claims 1 to 3, wherein the fluorescent dye is covalently bound to the fine particles.

5. The tissue-marking agent according to any one of claims 1 to 4, wherein a pH of the tissue-marking agent is in a range of 5.0 to 8.0.

6. The tissue-marking agent according to any one of claims 3 to 5, wherein the polysaccharide is at least one selected from the group consisting of hyaluronic acid, alginic acid, guar gum, xanthan gum, acacia gum, pullulan, dextran, and agarose, carrageenan, pectin, galactomannan, xanthan gum, gellan gum, curdlan, carboxymethyl cellulose, chitin/chitosan, and carrageenan.

7. The tissue-marking agent according to claim 6, wherein the polysaccharide comprises alginic acid, dextran, and/or hyaluronic acid.

8. The tissue-marking agent according to any one of claims 1 to 7, wherein the fluorescent dye is selected from the group consisting of indocyanine green, coumarin, rhodamine, xanthene, porphyrin, fluorescamine, fluorescein, and derivatives thereof.

9. The tissue-marking agent according to claim 8, wherein the fluorescent dye is indocyanine green or a derivative thereof.

10. The tissue-marking agent according to any one of claims 1 to 9, wherein the fine particles are selected from the group consisting of silica particles, polyester particles, polyurethane particles, silicon particles, polyethylene particles, polypropylene particles, polystyrene particles, polymethacrylate particles, titanium alloy particles, hydroxyapatite particles, cellulose particles, silk particles, alumina particles, zirconia particles, and titania particles.

11. The tissue-marking agent according to any one of claims 1 to 10, wherein the fine particles have an average particle diameter in a range of 1 nm or more to 2 µm or less.

12. The tissue-marking agent according to any one of claims 1 to 11, wherein the dispersion medium is at least one selected from the group consisting of physiological saline, phosphate buffer, acetate buffer, citrate buffer, prolamin buffer, carbon buffer, and purified water.

13. The tissue-marking agent according to any one of claims 1 to 12, wherein a concentration of the fine particles in the dispersion medium is in a range of 0.01 mg/mL or more to 1,000 mg/mL or less.

14. The tissue-marking agent according to any one of claims 1 to 13, wherein a concentration of the water-soluble viscous substance in the dispersion medium is in a range of 0.001 mass% or more to 20 mass% or less.

15. The tissue-marking agent according to any one of claims 1 to 14 for marking a localized area of a gastrointestinal tract.

16. Use of an aqueous composition for preparing a tissue-marking agent, wherein the aqueous composition comprises fine particles labelled with a fluorescent dye dispersed in an aqueous medium in which a water-soluble viscous substance is dissolved.

17. The use according to claim 16, wherein the water-soluble viscous substance comprises a polyhydric alcohol, a polyether or a salt thereof, a water-soluble protein, or a polysaccharide or a salt thereof.

18. The use according to claim 16, wherein the water-soluble viscous substance comprises a polysaccharide or a salt thereof.

19. The use according to any one of claims 16 to 18, wherein the fluorescent dye is covalently bound to the fine particles.

20. The use according to any one of claims 16 to 19, wherein a pH of the tissue-marking agent is in a range of 5.0 to 8.0.

21. The use according to any one of claims 18 to 20, wherein the polysaccharide is at least one selected from the group consisting of hyaluronic acid, alginic acid, guar gum, xanthan gum, acacia gum, pullulan, dextran, and agarose, carrageenan, pectin, galactomannan, xanthan gum, gellan gum, curdlan, carboxymethyl cellulose, chitin/chitosan, and carrageenan.

22. The use according to claim 21, wherein the polysaccharide comprises or is alginic acid, dextran, and/or hyaluronic acid.

23. The use according to any one of claims 16 to 22, wherein the fluorescent dye is selected from the group consisting of indocyanine green, coumarin, rhodamine, xanthene, porphyrin, fluorescamine, and derivatives thereof.

24. The use according to claim 23, wherein the fluorescent dye is indocyanine green or a derivative thereof.

25. The use according to any one of claims 16 to 24, wherein the fine particles are selected from the group consisting of silica particles, polyester particles, polyurethane particles, silicon particles, polyethylene particles, polypropylene particles, polystyrene particles, polymethacrylate particles, titanium alloy particles, hydroxyapatite particles, cellulose particles, silk particles, alumina particles, zirconia particles, and titania particles.

26. The use according to any one of claims 16 to 25, wherein the fine particles have an average particle diameter in a range of 1 nm or more to 2 µm or less.

27. The use according to any one of claims 16 to 26, wherein the dispersion medium is at least one selected from the group consisting of physiological saline, phosphate buffer, acetate buffer, citrate buffer, prolamin buffer, carbon buffer, and purified water.

28. The use according to any one of claims 16 to 27, wherein a concentration of the fine particles in the dispersion medium is in a range of 0.01 mg/mL or more to 1,000 mg/mL or less.

29. The use according to any one of claims 16 to 28, wherein a concentration of the water-soluble viscous substance in the dispersion medium is in a range of 0.001 mass% or more to 20 mass% or less.

30. The use according to any one of claims 16 to 29, wherein the tissue-marking agent is used for marking a localized area of a gastrointestinal tract.

31. An aqueous composition for use in marking tissue, wherein fine particles labelled with a fluorescent dye are dispersed in an aqueous medium in which a water-soluble viscous substance is dissolved.

32. The aqueous composition for use according to claim 31, wherein the water-soluble viscous substance comprises a polyhydric alcohol, a polyether or a salt thereof, a water-soluble protein, or a polysaccharide or a salt thereof.

33. The aqueous composition for use according to claim 31, wherein the water-soluble viscous substance comprises a polysaccharide or a salt thereof.

34. The aqueous composition for use according to any one of claims 31 to 33, wherein the fluorescent dye is covalently bound to the fine particles.

35. The aqueous composition for use according to any one of claims 31 to 34, wherein a pH of the aqueous composition is in a range of 5.0 to 8.0.

36. The aqueous composition for use according to any one of claims 31 to 35, wherein the polysaccharide is at least one selected from the group consisting of hyaluronic acid, alginic acid, guar gum, xanthan gum, acacia gum, pullulan, dextran, and agarose, carrageenan, pectin, galactomannan, xanthan gum, gellan gum, curdlan, carboxymethyl cellulose, chitin/chitosan, and carrageenan.

37. The aqueous composition for use according to claim 36, wherein the polysaccharide comprises or is alginic acid, dextran, and/or hyaluronic acid.

38. The aqueous composition for use according to any one of claims 31 to 37, wherein the fluorescent dye is selected from the group consisting of indocyanine green, coumarin, rhodamine, xanthene, porphyrin, fluorescamine, and derivatives thereof.

39. The aqueous composition for use according to claim 38, wherein the fluorescent dye is indocyanine green or a derivative thereof.

40. The aqueous composition for use according to any one of claims 31 to 39, wherein the fine particles are selected from the group consisting of silica particles, polyester particles, polyurethane particles, silicon particles, polyethylene particles, polypropylene particles, polystyrene particles, polymethacrylate particles, titanium alloy particles, hydroxyapatite particles, cellulose particles, silk particles, alumina particles, zirconia particles, and titania particles.

41. The aqueous composition for use according to any one of claims 31 to 40, wherein the fine particles have an average particle diameter in a range of 1 nm or more to 2 µm or less.

42. The aqueous composition for use according to any one of claims 31 to 41, wherein the dispersion medium is at least one selected from the group consisting of physiological saline, phosphate buffer, acetate buffer, citrate buffer, prolamin buffer, carbon buffer, and purified water.

43. The aqueous composition for use according to any one of claims 31 to 42, wherein a concentration of the fine particles in the dispersion medium is in a range of 0.01 mg/mL or more to 1,000 mg/mL or less.

44. The aqueous composition for use according to any one of claims 31 to 43, wherein a concentration of the water-soluble viscous substance in the dispersion medium is in a range of 0.001 mass% or more to 20 mass% or less.

45. The aqueous composition for use according to any one of claims 31 to 44, wherein the tissue-marking agent is used for marking a localized area of a gastrointestinal tract.

46. A tissue-marking method comprising: topically administering, for example, injecting the tissue-marking agent according to any of claims 1 to 15 or the aqueous composition according to any of claims 31 to 45 to a target site of tissue.

47. The tissue-marking agent according to any of claims 1 to 15 or the aqueous composition according to any of claims 31 to 45, comprising an antioxidant.

48. The tissue-marking agent or the aqueous composition according to claim 32, comprising the antioxidant at 0.02 mg/mL to 10 mg/mL.

49. The use according to any one of claims 16 to 30, wherein the tissue-marking agent comprises an antioxidant.

50. The use according to claim 34, wherein the tissue-marking agent comprises the antioxidant at 0.02 mg/mL to 10 mg/mL.
